(19) Europäisches Patentamt / European Patent Office / Office européen des brevets

(11) **EP 3 666 948 B1**

(12) **EUROPEAN PATENT SPECIFICATION**

(45) Date of publication and mention
of the grant of the patent:
**16.11.2022 Bulletin 2022/46**

(21) Application number: **18853798.9**

(22) Date of filing: **27.08.2018**

(51) International Patent Classification (IPC):
*D04H 1/56* (2006.01)  *D04H 3/16* (2006.01)
*D04H 3/007* (2012.01)  *D04H 3/016* (2012.01)
*B01D 39/16* (2006.01)  *A61L 15/24* (2006.01)
*A61L 15/42* (2006.01)  *A61K 9/70* (2006.01)
*A61L 15/44* (2006.01)

(52) Cooperative Patent Classification (CPC):
(C-Sets available)
**D04H 3/016; A61L 15/24; A61L 15/425;**
**B01D 39/1623; D04H 1/56; D04H 3/007;**
**D04H 3/16;** A61K 9/7007; A61L 15/44;
B01D 2239/0622; B01D 2239/1233;
B01D 2239/1258                         (Cont.)

(86) International application number:
**PCT/JP2018/031593**

(87) International publication number:
**WO 2019/049706 (14.03.2019 Gazette 2019/11)**

(54) **MELT-BLOWN NONWOVEN FABRIC AND MANUFACTURING METHOD THEREFOR**

SCHMELZGEBLASENER VLIESSTOFF UND HERSTELLUNGSVERFAHREN DAFÜR

NON-TISSÉ PRODUIT PAR EXTRUSION-SOUFFLAGE ET SON PROCÉDÉ DE FABRICATION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **08.09.2017 JP 2017173124**

(43) Date of publication of application:
**17.06.2020 Bulletin 2020/25**

(73) Proprietor: **Kuraray Co., Ltd.**
**Kurashiki-shi, Okayama 710-0801 (JP)**

(72) Inventors:
• **OKAMOTO, Tetsuya**
**Saijo-shi**
**Ehime 793-8585 (JP)**

• **SHIROTANI, Yasuhiro**
**Saijo-shi**
**Ehime 793-8585 (JP)**

(74) Representative: **Vossius & Partner**
**Patentanwälte Rechtsanwälte mbB**
**Siebertstrasse 3**
**81675 München (DE)**

(56) References cited:
**JP-A- 2003 504 523    JP-A- 2011 231 163**
**JP-A- 2011 256 490    JP-A- 2015 190 081**

(52) Cooperative Patent Classification (CPC): (Cont.)

C-Sets
**A61L 15/24, C08L 23/00**

C-Sets
**A61L 15/24, C08L 23/00**

**Description**

FIELD OF THE INVENTION

The present invention relates to a melt-blown (MB) nonwoven fabric including fibers containing a cyclic olefinic resin, the fabric in which alienated large-diameter fibers are reduced, and a production method of the melt-blown (MB) nonwoven fabric.

BACKGROUND OF THE INVENTION

A cyclic olefinic resin is molded by various methods because of its features such as high transparency, high heat resistance, chemical resistance, low elution of impurities, and low adsorption, and is used for medical applications such as chemical packaging materials and test containers, optical applications such as lenses and optical films, electronic device applications, etc.

For example, Patent Document 1 (JP Laid-open Patent Publication No. 2001-210549) describes an electret formed from a resin composition containing a cyclic hydrocarbon polymer, and also indicates that the electret can be in the form of a film, a sheet, fibers, a nonwoven fabric, or the like, and electretization of a nonwoven fabric is preferably achieved by a melt-blowing method.

CONVENTIONAL ART DOCUMENT

PATENT DOCUMENT

[Patent Document 1] JP Laid-open Patent Publication No. 2001-210549
[Patent Document 2] WO 01/02635 A1
[Patent Document 3] US 2011/0259818

SUMMARY OF THE INVENTION

PROBLEMS TO BE SOLVED BY THE INVENTION However, Patent Document 1 merely describes as a production method of a melt-blown nonwoven fabric the conditions of a general melt-blowing method, and films were merely produced in Examples. Patent Document 2 relates to a method of producing microfiber nonwovens that contain at least one cycloolefin polymer by means of a melt-blow method and to articles produced according to this method. Patent Document 3 discloses filter media for liquid purification, which can remove metal compounds or metal ions contained in polishing or washing liquids such as alkali, acid solution or ultra-pure water used for silicon wafers of semiconductors.

Therefore, an object of the present invention is to provide a melt-blown nonwoven fabric that includes fibers containing a cyclic olefinic resin, the fabric in which intermingling of thick fibers is suppressed; a filter provided with the melt-blown nonwoven fabric; a support for transdermal administration sheet, and a transdermal administration sheet (particularly, a transdermal absorption preparation (percutaneous absorbent) or a skin patch).

Another object of the present invention is to provide a production method of a melt-blown nonwoven fabric including fibers containing a cyclic olefinic resin, the method being capable of suppressing intermingling of thick fibers.

MEANS FOR SOLVING THE PROBLEMS

The inventors of the present invention have conducted thorough research for achieving the above objects, and, first, have found that (i) a melt-blowing method applied to a cyclic olefinic resin induces a phenomenon called as "mechanochemical reaction" in which degradation of the main chain of the resin by mechanical energy during kneading of the resin forms new bonds from the degraded double bonds, resulting in gelation of fibers, whereby bead-like lumps called shots are generated in an MB nonwoven fabric made from the cyclic olefinic resin, and that even when shots themselves are not intermingled in the MB nonwoven fabric, fibers derived from the mechanochemical reaction and having thicker diameters than fibers having acceptable diameters are intermingled as defects in the MB nonwoven fabric. The inventors of the present invention also have found that (ii) in the melt-blowing method, where mechanical energy (particularly, compressive stress) acts on the resin during the process such as kneading of the resin in an extruder, or discharge of the resin to a die, mechanochemical reaction of the resin easily occurs, and have then conducted further research. As a result, the inventors of the present invention have finally found that (iii) an MB nonwoven fabric in which generation of thick fibers is suppressed can be obtained by using a method in which compression of the resin is reduced as small as possible, for example, by decreasing the viscosity of a resin melt before introduction to a kneading portion and/or by decreasing the ratio (Q/N) of a discharge amount (Q) of a resin-kneaded material to a rotation speed (N) of a screw, and have then completed the present invention.

The present invention relates to a production method of a melt-blown nonwoven fabric according to claim 1 and to a melt-blown nonwoven fabric according to claim 4.

EFFECT OF THE INVENTION

In the MB nonwoven fabric according to the present invention, even though the MB nonwoven fabric includes fibers containing the cyclic olefinic resin which causes a specific side reaction, it is possible to obtain an MB nonwoven fabric in which intermingling of thick fibers is suppressed so as to provide a filter and a transdermal administration sheet (particularly, a transdermal absorption preparation or a skin patch) in which the MB nonwoven fabric is used.

According to the present invention, even when a nonwoven fabric is produced by a melt-blowing method, occurrence of "mechanochemical reaction" which is specific to the cyclic olefinic resin can be suppressed by setting the viscosity of the resin melt within a specific range before the resin melt is introduced to the kneading portion, and, as a result, it is possible to provide a production method of a melt-blown nonwoven fabric in which intermingling of thick fibers is suppressed.

BRIEF DESCRIPTION OF THE DRAWINGS

The present invention will be more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the figures,

Fig. 1 is a schematic cross-sectional view showing an apparatus used for producing an MB nonwoven fabric according to an embodiment of the present invention;
Fig. 2 is an SEM image (magnification: 300) of an MB nonwoven fabric obtained in Example 1;
Fig. 3 is an SEM image (magnification: 300) of an MB nonwoven fabric obtained in Example 2;
Fig. 4 is an SEM image (magnification: 300) of an MB nonwoven fabric obtained in Example 3;
Fig. 5 is an SEM image (magnification: 300) of an MB nonwoven fabric obtained in Example 4;
Fig. 6 is an SEM image (magnification: 300) of an MB nonwoven fabric obtained in Comparative Example 1.

DESCRIPTION OF THE EMBODIMENTS

Melt-blown Nonwoven Fabric

A melt-blown nonwoven fabric according to the present invention includes fibers containing a cyclic olefinic resin, wherein, among fiber diameter data at 100 points that are randomly selected from a scanning electron microscope (SEM) image taken in a magnification of 1,000, and ordered from a smallest fiber diameter to a largest fiber diameter and divided into quartiles, an average value of fiber diameter data having a larger than a third quartile is equal to or less than 30 $\mu$m.

The term "quartiles" refer to values by each of which the data in ascending order are divided into four equal groups. From the smallest, the value of the data at 1/4 is referred to as a first quartile, the value of the data at 2/4 is referred to as a second quartile (median), and the value of the data at 3/4 is referred to as a third quartile.

The MB nonwoven fabric according to the present invention has a reduced number of thick fibers derived from mechanochemical reaction. Among 100 points randomly selected from a scanning electron microscope image of the nonwoven fabric taken in a magnification of 1,000, fibers having a fiber diameter larger than a third quartile of fiber diameter data, i.e., 76th to 100th pieces of data, have an average fiber diameter (Db) of equal to or less than 30 $\mu$m, preferably equal to or less than 20 $\mu$m, and more preferably equal to or less than 16 $\mu$m. Decreasing in fiber diameter average value in the data of a fiber diameter larger than the third quartile of the fiber diameter can enhance the denseness of the nonwoven fabric, so that the nonwoven fabric can have good texture and can also be pleasant to touch. The lower limit of the average fiber diameter Db is not particularly limited to the specific one, and may be, for example, the average fiber diameter of the fibers constituting the nonwoven fabric.

The MB nonwoven fabric according to the present invention may have an average fiber diameter of the fibers constituting the nonwoven fabric of, for example, 1 to 15 $\mu$m, preferably 1 to 12 $\mu$m, more preferably 1 to 10 $\mu$m, and further preferably 1 to 8 $\mu$m from the viewpoint of improving the denseness of the nonwoven fabric. The average fiber diameter refers to the average value of all the measured data of fiber diameter, and is a value measured in accordance with a method described in the below-described Examples.

The MB nonwoven fabric according to the present invention has a CV value of the average fiber diameter of the fibers constituting the nonwoven fabric of, equal to or less than 110%, preferably equal to or less than 105% from the viewpoint of improving the denseness and the uniformity of texture of the nonwoven fabric. The lower limit value of the CV value is not particularly limited to the specific one, and the CV value may be equal to or greater than 50%. The CV value of the average fiber diameter refers to the ratio of the standard deviation of the measured fiber diameter data with respect to the average fiber diameter, is an index representing variations in a fiber diameter distribution, and is a value measured in accordance with a method described in the below-described Examples.

In the MB nonwoven fabric according to the present invention, from the viewpoint of improving the denseness and the uniformity of texture of the nonwoven fabric, the ratio of the average fiber diameter (Db) with respect to an average fiber diameter (Dc), as Db/Dc, may be, for example, equal to or less than 4.0, preferably equal to or less than 3.5 wherein the Db is the average value of data (76th to 100th pieces of data) having a fiber diameter larger than a third quartile of fiber diameter data at 100 points randomly selected from a scanning electron microscope image of the nonwoven fabric taken in a magnification of 1,000, and Dc is the average value of data (26th to 75th pieces of data) having a fiber diameter larger than a first quartile and smaller than the third quartile of the fiber diameter data. In addition, from the viewpoint of uniformity, Db/Dc is preferably equal to or greater than 1.

The fibers constituting the MB nonwoven fabric according to the present invention are fibers containing a cyclic olefinic resin. For example, a resin composition forming the fibers may include the cyclic olefinic resin in an amount of 50 mass% or greater, preferably in an amount of 80 mass% or greater, more preferably in an amount of 90 mass% or greater, and further preferably in an amount of 98 mass% or greater.

The MB nonwoven fabric according to the present invention is not particularly limited to the specific one as long as the effects of the present invention can be achieved, and the MB nonwoven fabric according to the present invention may contain a thermoplastic resin in addition to the cyclic olefinic resin described later. Examples of the thermoplastic resin may include chain polyolefinic resins (acyclic olefinic resins) (for example, polyethylene-series resins and polypropylene-series resins) and polyester-series resins (for example, polyethylene terephthalate-series resins and polybutylene terephthalate-series resins).

From the viewpoint of low elution, the cyclic olefinic resin described later may form at least a part of the surfaces of the fibers constituting the MB nonwoven fabric according to the present invention.

The fibers constituting the MB nonwoven fabric according to the present invention are not particularly limited to the specific one as long as the effects of the present invention can be achieved, and the fibers may be composite fibers, and may be, for example, core-sheath fibers, side-by-side fibers, or sea-island fibers.

The basis weight of the MB nonwoven fabric according to the present invention can be determined as appropriate according to application of the MB nonwoven fabric. Although the basis weight is not particularly limited to the specific one, for example, from the viewpoint of weight reduction and denseness, the basis weight may be, for example, about 10 to 50 $g/m^2$, preferably about 10 to 45 $g/m^2$ and more preferably about 10 to 40 $g/m^2$. The basis weight is a value measured in accordance with a method described in the below-described Examples.

The thickness of the MB nonwoven fabric according to the present invention can be determined as appropriate according to application of the MB nonwoven fabric. Although the thickness is not particularly limited to the specific one, for example, from the viewpoint of weight reduction and feeling, the thickness may be, for example, about 0.10 to 1.00 mm, preferably about 0.10 to 0.85 mm and more preferably about 0.10 to 0.70 mm. The thickness is a value measured in accordance with a method described in the below-described Examples.

The air permeability of the MB nonwoven fabric according to the present invention can be determined as appropriate according to application of the MB nonwoven fabric. Although the air permeability is not particularly limited to the specific one, for example, from the viewpoint of denseness, the air permeability may be, for example, about 10 to 550 $cm^3/cm^2 \cdot s$, preferably about 10 to 500 $cm^3/cm^2 \cdot s$ and more preferably about 10 to 400 $cm^3/cm^2 \cdot s$. The air permeability is a value measured in accordance with a method described in the below-described Examples.

The MB nonwoven fabric according to the present invention preferably has excellent denseness regardless of a small thickness, and, for example, the ratio (B/A) of the air permeability B ($cm^3/cm^2 \cdot s$) with respect to the thickness A (mm) of the nonwoven fabric may satisfy $80 \leq B/A \leq 800$, preferably $90 \leq B/A \leq 500$, and more preferably satisfy $100 \leq B/A \leq 400$.

The MB nonwoven fabric according to the present invention can be used for various applications such as medical, beauty, and hygiene materials, industrial materials, daily commodities, and clothing. Among them, the MB nonwoven fabric according to the present invention is suitable for use for various filters such as a filter for filtrating a liquid such as water and an air filter (for example, an air filter for a clean room), a transdermal administration sheet (for example, a transdermal absorption preparation or a skin patch), etc., and is particularly suitable for use for a filter for filtrating a liquid and a transdermal administration sheet (for example, a transdermal absorption preparation or a skin patch) since the MB nonwoven fabric according to the present invention is excellent in low elution of impurities.

When the MB nonwoven fabric according to the present invention is used for various filters, a filter collection efficiency measured in accordance with JIS T 8151 may be preferably equal to or greater than 85%, more preferably equal to or greater than 88%, and even more preferably equal to or greater than 90%. In addition, a filter pressure loss measured in accordance with JIS T 8151 may be preferably equal to or less than 15 Pa, more preferably equal to or less than 13 Pa, and even more preferably equal to or less than 10 Pa. The collection efficiency and the pressure loss are values measured in accordance with a method described in the below-described Examples.

The MB nonwoven fabric according to the present invention is very useful for an air filter achieving low pressure loss and high collection efficiency. In addition, since the cyclic olefinic resin is a polymer with low elution nature approved by the FDA (U.S. Food and Drug Administration), the MB nonwoven fabric can be expected to be applied to a filter for filtrating a liquid used for water purification and so on. Where the MB nonwoven fabric according to the present invention is used for a filter for filtrating a liquid, the filter used for water purification and so on, the MB nonwoven fabric may have a decreased fiber diameter, and, for example, the average fiber diameter of the MB nonwoven fabric may be 1 to 8 $\mu$m, and preferably 1 to 7 $\mu$m.

Furthermore, as a medical application and beauty application, the MB nonwoven fabric according to the present invention is suitable for use for a transdermal administration sheet (preferably, a transdermal absorption preparation or a skin patch) releasing an active ingredient (for example, a pharmacological (pharmaceutical) or cosmetic active ingredient, preferably a pharmacological active ingredient), since the MB nonwoven fabric according to the present invention has advantageous nature in which impurity elution is low and active ingredients such as medicaments barely transfer to the nonwoven fabric. The transdermal administration sheet can include at least an active ingredient and the MB nonwoven fabric according to the present invention as a support. Specifically, in the transdermal administration sheet, the MB nonwoven fabric according to the present invention may be used as a support for holding a liquid containing an active ingredient, or may be used as a support (backing) for supporting a semi-solid or solid material containing an active ingredient.

Cyclic Olefinic Resin

The cyclic olefinic resin is a polymer or a copolymer that includes structural units derived from a cyclic olefin in the main chain. This cyclic olefin is an unsaturated hydrocarbon compound having at least one olefinic double bond in a cyclic hydrocarbon structure, typified by norbornene, dicyclopentadiene, and tetracyclododecene. The cyclic olefin can be introduced to polymeric structure by using the cyclic olefins as a monomer. Examples of the cyclic olefinic resin include, an addition polymer of a cyclic olefin or a hydrogenated compound thereof; an addition copolymer of a cyclic olefin and an $\alpha$-olefin or a hydrogenated compound thereof; and a ring-opening (co)polymer of a cyclic olefin or a hydrogenated compound thereof from the production method of the cyclic olefinic resin. The cyclic olefinic resin in the present invention may be, for example, an addition copolymer of a cyclic olefin and an $\alpha$-olefin from the viewpoint of both characteristics and cost.

The cyclic olefin may include, for example: a monocyclic olefin such as cyclopentene, cyclohexene, cyclooctene, cyclopentadiene, and 1,3-cyclohexadiene; a bicyclic olefin such as bicyclo[2.2.1]hept-2-ene (norbornene), 5-methyl-bicyclo[2.2.1]hept-2-ene, 5,5-dimethyl-bicyclo[2.2.1]hept-2-ene, 5-ethyl-bicyclo[2.2.1]hept-2-ene, 5-butyl-bicyclo[2.2.1]hept-2-ene, 5-ethylidene-bicyclo[2.2.1]hept-2-ene, 5-hexyl-bicyclo[2.2.1]hept-2-ene, 5-octyl-bicyclo[2.2.1]hept-2-ene, 5-octadecyl-bicyclo[2.2.1]hept-2-ene, 5-methylidene-bicyclo[2.2.1]hept-2-ene, 5-vinyl-bicyclo[2.2.1]hept-2-ene, and 5-propenyl-bicyclo[2.2.1]hept-2-ene; a tricyclic olefin such as tricyclo[4.3.0.1$^{2,5}$]deca-3,7-diene (dicyclopentadiene), tricyclo[4.3.0.1$^{2,5}$]dec-3-ene, tricyclo[4.4.0.1$^{2,5}$]undeca-3,7-diene or tricyclo[4.4.0.1$^{2,5}$]undeca-3,8-diene or tricyclo[4.4.0.1$^{2,5}$]undec-3-ene as a partial hydrogenated product thereof (or a adduct of cyclopentadiene and cyclohexene), 5-cyclopentyl-bicyclo[2.2.1]hept-2-ene, 5-cyclohexyl-bicyclo[2.2.1]hept-2-ene, 5-cyclohexenyl-bicyclo[2.2.1]hept-2-ene, and 5-phenyl-bicyclo[2.2.1]hept-2-ene; a tetracyclic olefin such as tetracyclo[4.4.0.1$^{2,5}$.1$^{7,10}$]dodec-3-ene (tetracyclododecene), 8-methyl-tetracyclo[4.4.0.1$^{2,5}$.1$^{7,10}$]dodec-3-ene, 8-ethyl-tetracyclo[4.4.0.1$^{2,5}$.1$^{7,10}$]dodec-3-ene, 8-methylidene-tetracyclo[4.4.0.1$^{2,5}$.1$^{7,10}$]dodec-3-ene, 8-ethylidene-tetracyclo[4.4.0.1$^{2,5}$.1$^{7,10}$]dodec-3-ene, 8-vinyl-tetracyclo[4.4.0.1$^{2,5}$.1$^{7,10}$]dodec-3-ene, and 8-propenyl-tetracyclo[4.4.0.1$^{2,5}$.1$^{7,10}$]dodec-3-ene; a polycyclic olefin such as 8-cyclopentyl-tetracyclo[4.4.0.1$^{2,5}$.1$^{7,10}$]dodec-3-ene, 8-cyclohexyl-tetracyclo[4.4.0.1$^{2,5}$.1$^{7,10}$]dodec-3-ene, 8-cyclohexenyl-tetracyclo[4.04.0.1$^{2,5}$.1$^{7,10}$]dodec-3-ene, 8-phenyl-cyclopentyl-tetracyclo[4.4.0.1$^{2,5}$.1$^{7,10}$]dodec-3-ene, tetracyclo[7.4.1$^{3,6}$.0$^{1,1}$.0$^{2,7}$]tetradeca-4,9,11,13-tetraene(1,4-methano-1,4,4a,9a,-tetrahydrofluorene), tetracyclo[8.4.1$^{4,7}$.0$^{1,10}$.0$^{3,8}$]pentadeca-5,10,12,14-tetraene(1,4-methano-1,4,4a,5,10,10a-hexahydroanthracene), pentacyclo[6.6.1.1$^{3,6}$.0$^{2,7}$.0$^{9,14}$]-4-hexadecene, pentacyclo[6.5.1.1$^{3,6}$.0$^{2,7}$.0$^{9,13}$]-4-pentadecene, pentacyclo[7.4.0.0$^{2,7}$.1$^{3,6}$.1$^{10,13}$]-4-pentadecene, heptacyclo[8.7.0.1$^{2,9}$.1$^{4,7}$.1$^{11,17}$.0$^{3,8}$.0$^{12,16}$]-5-eicosene, heptacyc10[8.7.0.1$^{2,9}$.0$^{3,8}$.1$^{4,7}$.0$^{12,17}$.1$^{13,16}$]-14-eicosene, and a tetramer of cyclopentadiene. These cyclic olefins may be used singly or in combination of two or more of the cyclic olefins.

Specific examples of an $\alpha$-olefin copolymerizable with a cyclic olefin may include an $\alpha$-olefin having 2 to 20 carbon atoms, preferably 2 to 8 carbon atoms, such as ethylene, propylene, 1-butene, 1-pentene, 1-hexene, 3-methyl-1-butene, 3-methyl-1-pentene, 3-ethyl-1-pentene, 4-methyl-1-pentene, 4-methyl-1-hexene, 4,4-dimethyl-1-hexene, 4,4-dimethyl-1-pentene, 4-ethyl-1-hexene, 3-ethyl-1-hexene, 1-octene, 1-decene, 1-dodecene, 1-tetradecene, 1-hexadecene, 1-octadecene, and 1-eicosene. These $\alpha$-olefins may be used singly or in combination of two or more of the $\alpha$-olefins.

The polymerization method for a cyclic olefin or a cyclic olefin and an $\alpha$-olefin and the hydrogenation method are not particularly limited to the specific one, and known methods can be used.

The cyclic olefinic resin used in the present invention is preferably a copolymer in which at least norbornene is used as a cyclic olefin and which includes at least norbornene units, and is particularly preferably a copolymer of ethylene and norbornene.

For example, in the copolymer of ethylene and norbornene, from the viewpoint of processability and elution property, the mass ratio of norbornene units to the total of ethylene units and the norbornene units may be 60 to 99 mass% and preferably 63 to 90 mass%.

Production Method of Melt-blown Nonwoven Fabric

The production method of the MB nonwoven fabric according to the present invention includes at least:

> a melting step of melting a resin composition containing at least the cyclic olefinic resin by heating in a supply portion to obtain a resin melt;
> a kneading step of introducing the resin melt to a kneading portion and kneading the resin melt in the kneading portion by rotation of a screw under heating to obtain a resin-kneaded material;
> a discharging step of discharging the resin-kneaded material with air from a nozzle; and
> a collecting step of collecting a fibrous material discharged from the nozzle on a collection surface to obtain a web.

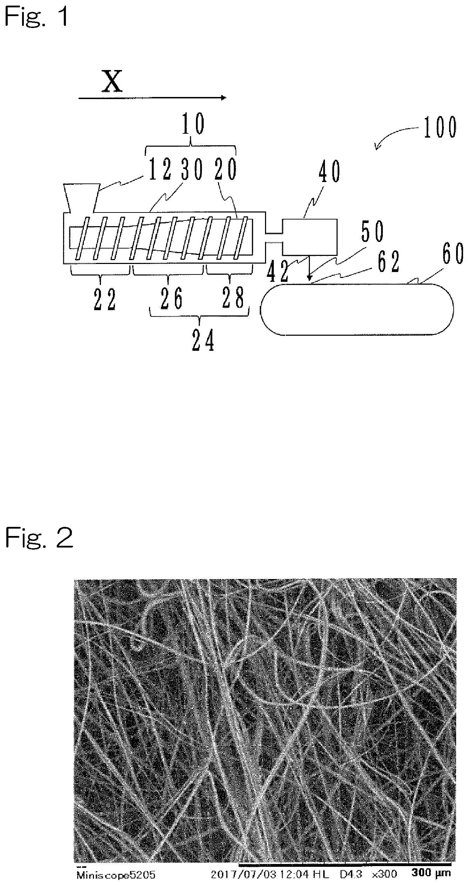

Fig. 1 is a schematic cross-sectional view showing an apparatus 100 used for producing an MB nonwoven fabric according to an embodiment of the present invention. As shown in Fig. 1, the apparatus 100 is provided with at least an extruder 10, a die 40, and a collecting member 60. The extruder 10 includes at least a cylinder 30 and a screw 20 that rotates within the cylinder 30. The screw 20 includes a supply portion 22 in which a solid resin is supplied, and a kneading portion 24 in which the resin supplied from the supply portion 22 is kneaded. In Fig. 1, the resin composition containing the cyclic olefinic resin is heated and melted in the supply portion 22 in the screw 20 of the extruder 10 and introduced to the kneading portion 24.

In Fig. 1, the kneading portion 24 includes a compressing portion 26 and a weighing portion 28. For example, a solid resin composition (solid resin) introduced from a hopper 12 is sent toward the die 40 from the supply portion 22 to the kneading portion 24 within the cylinder 30 by rotation of the screw 20.

Typically, in the screw 20, the supply portion 22 has grooves with uniform depth (Hp), and the compressing portion 26 has grooves that gradually get shallower in a flow direction X. The depth of the grooves of the screw 20 is the smallest at the weighing portion 28, and the weighing portion 28 has grooves with uniform depth (Hm) (Hp>Hm).

In the kneading portion 24 in the screw 20, the grooves of the screw 20 get shallower in the flow direction X, and thus mechanical energy acting on the resin melt between the screw 20 and an inner wall of the cylinder 30 is increased as the resin melt proceeds in the flow direction X. The resin-kneaded material through the kneading portion 24 is then introduced to the die 40. Then, the resin-kneaded material is discharged from a nozzle 42, and a discharged fibrous material 50 is collected on a collection surface 62, whereby a nonwoven fabric is produced.

Melting Step

As shown in Fig. 1, in the melting step, the resin composition containing at least the cyclic olefinic resin is transferred by rotation of the screw 20 in the supply portion 22 of the screw 20 provided within the cylinder 30, so as to be melted by heating using a known heating means such as a heater installed on the cylinder 30. In the supply portion 22, the solid resin supplied from the hopper 12 forms solid beds between the screw 20 and the cylinder 30 and moves in the flow direction X.

According to the typical method, the solid beds are not destroyed in the supply portion but destroyed in the kneading portion 24 to form a resin melt. In contrast, according to the present invention, by increasing the heating temperature in the supply portion 22 to about a temperature in which the solid beds are destroyed, a resin melt having a specific viscosity can be formed in the supply portion 22. The above description is given with a single-screw extruder as an example of the extruder 10, but a known extruder such as a single-screw extruder and a multi-screw extruder (with two or more screws) can be used as the extruder 10.

The resin composition is conventionally introduced to the kneading portion as a solid or in a state of containing a solid. However, according to the production method of the MB nonwoven fabric according to the present invention, since the resin composition is made in advance into a resin melt having a specific low viscosity prior to heating and kneading in the kneading portion, it is possible to decrease mechanical energy acting on the resin melt, for example, shear stress caused by rotation of the screw in the kneading step as well as compressive stress to the resin melt supplied to the die and/or discharged from the nozzle. Accordingly, it is possible to suppress "mechanochemical reaction" which is characteristically observed in the cyclic olefinic resin, and, as a result, generation of thick fibers can be suppressed.

The heating temperature in the supply portion can be selected as appropriate according to the viscosity properties, the thermal decomposition temperatures, etc., of the cyclic olefinic resin and other thermoplastic resin contained in the resin composition. The heating temperature may be, for example, 200 to 400°C, preferably 250 to 390°C and more preferably 300 to 380°C. By increasing the heating temperature in the supply portion, it is possible to decrease a viscosity $\eta$ of the resin melt.

The viscosity $\eta$ of the resin melt at the heating temperature in the supply portion is 80 to 200 poise, and may be preferably 85 to 195 poise and more preferably 90 to 190 poise (1 poise = 0.1 Pa.s).

The viscosity is a value measured in accordance with a method described in the below-described Examples. The resin melt adjusted to a predetermined viscosity in the supply portion is introduced to the kneading portion.

Kneading Step

The resin melt that is melted in advance in the supply portion 22 is then introduced to the kneading portion 24, and kneaded by rotation of the screw 20 under heating in the kneading portion 24 to obtain a resin-kneaded material. By decreasing the viscosity of the resin melt in advance in a supply step, compressive stress acting on the resin-kneaded material when the resin-kneaded material is introduced to the die can be reduced in the kneading step.

The screw may satisfy the formula $(Q \times \eta)/N$ of, for example, 20 to 100, preferably 30 to 90 and more preferably 40 to 80, wherein the formula is the product of the viscosity $\eta$ (poise) of the resin melt and the ratio Q/N, i.e., the ratio of a discharge amount Q (kg/hr) of the resin-kneaded material in the below-described discharging step with respect to a rotation speed N (rpm) of the screw. The $(Q \times \eta)/N$ is a parameter, for mechanical energy given to the resin by rotation of the screw, in which the viscosity of the resin when being kneaded is reflected. By setting the $(Q \times \eta)/N$ within the above range, it is possible to decrease mechanical energy, particularly, compressive stress, given to the resin, and "mechanochemical reaction" which occurs in the cyclic olefinic resin can be suppressed.

From the viewpoint of decreasing mechanical energy, particularly, compressive stress, given to the resin, the ratio Q/N may be 0.1 to 2.0, preferably 0.2 to 1.5 and more preferably 0.3 to 1.0, wherein the ratio is the ratio of the discharge amount Q (kg/hr) of the resin-kneaded material in the below-described discharging step with respect to the rotation speed N (rpm) of the screw. The Q/N refers to a processing amount of the resin per rotation speed of the screw, and mechanical energy received by the resin in the kneading portion can be reduced by setting this value within the above range.

From the viewpoint of reducing the viscosity of the resin-kneaded material and decreasing the compressive stress given to the resin-kneaded material when the resin-kneaded material is supplied to the die, the ratio (L/D) of the length (L) in the axial direction of the screw with respect to the diameter (D) of the screw may be 15 to 40, preferably 18 to 40 and more preferably 20 to 40.

The heating temperature in the kneading portion can be selected as appropriate according to the viscosity properties, the thermal decomposition temperatures, etc., of the cyclic olefinic resin and other thermoplastic resin contained in the resin composition. From the viewpoint of reducing the viscosity of the resin-kneaded material, the heating temperature may be, for example, 230 to 400°C, preferably 250 to 390°C and more preferably 300 to 380°C.

Discharging Step

The obtained resin-kneaded material is introduced to the die 40, which includes the nozzle 42 for discharging the resin-kneaded material and air jet grooves (not shown) provided at both sides of the nozzle 42, and discharged from the nozzle 42 with air that is jetted out from the air jet grooves.

The hole diameter in the nozzle is not particularly limited to the specific one as long as fibers having a predetermined fiber diameter are obtained, and the hole diameter in the nozzle may be, for example, 0.01 to 1.0 mm, preferably 0.05 to 0.8 mm and more preferably 0.1 to 0.5 mm.

The heating temperature, that is, the spinning temperature, in the die, can be selected as appropriate according to the viscosity properties, the thermal decomposition temperatures, etc., of the cyclic olefinic resin and other thermoplastic resin contained in the resin composition. From the viewpoint of adjustment of the discharge amount of the resin-kneaded material, the spinning temperature may be, for example, 300 to 400°C, preferably 320 to 395°C and more preferably 350 to 390°C.

Collecting Step

Then, the discharged fibrous material 50 discharged from the nozzle 42 is collected on the collection surface 62 of the collecting member 60 to obtain a web. The collecting member 60 is not particularly limited to the specific one as long as the collecting member 60 is a collecting member used generally when an MB nonwoven fabric is produced. The collecting member 60 may be a rotating roll or may be a conveyor belt. For example, the collecting member 60 may be a conveyor belt that is circulated in one direction as shown in Fig. 1, and the discharged fibrous material 50 is collected on the collection surface 62, and an MB nonwoven fabric is continuously formed as the conveyor belt rotates.

Electrically Charging Step

The MB nonwoven fabric according to the present invention used for various filters may be subjected to electrically charging treatment (treatment that imparts electrical chargeability) in order to further improve the collecting performance of the MB nonwoven fabric. The term "electrically charged" refers to a state where the MB nonwoven fabric is charged with electricity, and the surface charge density (value obtained by dividing an amount of electric charge measured using a Faraday cage [electrostatic charge meter] by a measurement area) of the MB nonwoven fabric is preferably equal to or greater than $1.0 \times 10^{-10}$ coulomb/cm$^2$, more preferably equal to or greater than $1.5 \times 10^{-10}$ coulomb/cm$^2$, and further preferably equal to or greater than $2.0 \times 10^{-10}$ coulomb/cm$^2$.

As the method for imparting electrical chargeability to the MB nonwoven fabric, there may be mentioned appropriate electretization treatments such as a method of imparting electric charge by friction or contact, a method of applying active energy rays (for example, electron beam, ultraviolet ray, X-ray, etc.), a method using gas discharge such as corona discharge and plasma, a method using a high electric field, and a hydrocharging method using a polar solvent such as water. Among them, a corona discharge method and a hydrocharging method are preferable since high electrical chargeability is achieved with relatively low electric power.

An apparatus and conditions used for the corona discharge method are not particularly limited to the specific one. The corona discharge method can be carried out, for example, using a DC high voltage stabilized power supply, for example, under the conditions of linear distance between electrodes to which a voltage is applied: 5 to 70 mm (preferably 10 to 50 mm), applied voltage: -50 to -10 kV and/or 10 to 50 kV (preferably -40 to -20 kV and/or 20 to 40 kV), temperature: normal temperature (20°C) to 100°C (preferably 30 to 80°C), and treatment time: 0.1 to 20 seconds (preferably 0.5 to 10 seconds).

In the hydrocharging method, the MB nonwoven fabric is charged, for example, by spraying a polar solvent such as water and an organic solvent (preferably water from the viewpoint of productivity of waste water treatment, etc.), or by vibrating the MB nonwoven fabric while spraying the polar solvent. The pressure of the polar solvent to be impinged on the MB nonwoven fabric is preferably 0.1 to 5 MPa and more preferably 0.5 to 3 MPa, and the suction pressure from the lower side is preferably 500 to 5,000 mmH$_2$O and more preferably 1,000 to 3,000 mmH$_2$O. The hydrocharging period is preferably 0.01 to 5 seconds and more preferably 0.02 to 1 second. After the hydrocharging method is performed, the charged MB nonwoven fabric is preferably dried at a temperature of, for example, 40 to 100°C and preferably 50 to 80°C.

EXAMPLES

The present invention will be described in more detail below by means of examples, but the present invention is not limited to these examples in any manner. In the following Examples and Comparative Example, various physical property values were measured by the following methods.

Viscosity (poise) of Resin Melt

Viscosities of cyclic olefinic resins used in Examples and Comparative Example were measured by setting the measured temperature to the temperatures in a supply portion in Examples and Comparative Example using Toyo Seiki Capilograph Type 1B under the condition of shear rate r = 1200 sec$^{-1}$. Each of the viscosities measured under this condition is regarded as the viscosity ($\eta$) of a resin melt introduced to a kneading portion.

Average Fiber Diameter, Average Fiber Diameter of Fiber Diameters Larger Than Third Quartile

An MB nonwoven fabric fiber structure was observed using a scanning electron microscope. The fiber diameters of 100 fibers randomly selected from an electron micrograph taken in a magnification of 1,000 were measured, and, from the fiber diameter distribution of the measured fiber diameters, an average fiber diameter, a CV value, an average fiber diameter Dc of fibers having a fiber diameter larger than a first quartile and smaller than a third quartile, and an average fiber diameter Db of fibers having a fiber diameter larger than the third quartile were obtained.

Basis Weight

A basis weight (g/m²) of an MB nonwoven fabric was measured in accordance with JIS L 1913 "Test methods for nonwovens" 6.2.

Thickness

A thickness of an MB nonwoven fabric was measured in accordance with JIS L 1913 "Test methods for nonwovens" 6.1.

Air Permeability

An air permeability (cm³/cm²·s) was measured by a Frazier type method in accordance with JIS L 1096 "Testing methods for woven and knitted fabrics" 8.26.

Collecting Performance

Collection efficiency (%) and pressure loss (Pa)

In accordance with JIS T 8151, a piece having a size with a diameter of 11 cm was cut out of a charged nonwoven fabric and the cut-out piece was set on a sample stage having a filtration portion with a diameter of 8.6 cm (filtration area: 58.1 cm²), and the collection efficiency (%) and the pressure loss (Pa) were measured when NaCl particles (average particle diameter: 0.1 $\mu$m) were filtrated through the filtration portion at an air volume of 20 L/min and a face velocity of 5.7 cm/sec.

Example 1

An ethylene-norbornene copolymer (trade name "TOPAS" 5013, manufactured by Polyplastics Co., Ltd.) having a norbornene content of 75 mass%, as a cyclic olefinic resin, was heated and melted at 360°C in the supply portion to obtain a resin melt, and the obtained resin melt was introduced to the kneading portion. The viscosity ($\eta$) of the resin melt introduced to the kneading portion was 100 poise. In the kneading portion, the resin melt was kneaded at a screw rotation speed (N) of 5.0 rpm under heating at 380°C to obtain a resin-kneaded material. The obtained resin-kneaded material was supplied to a die having a nozzle with a nozzle single hole diameter (diameter) of 0.15 mm, the ratio of nozzle single hole length/nozzle single hole diameter = 10, and a nozzle hole pitch of 0.75 mm, and the resin-kneaded material was discharged from the nozzle at a spinning temperature of 380°C in a discharge amount (Q) of 2.2 kg/hr while hot air having a temperature of 340°C was blown at an air volume of 15 Nm³/min per 1 m of nozzle width. The fibrous material discharged from the nozzle was collected on a collection net to obtain a cyclic olefinic melt-blown nonwoven fabric having a basis weight of 34.2 g/m² and a thickness of 0.430 mm. A magnified electron micrograph of the obtained melt-blown nonwoven fabric is shown in Fig. 2.

Example 2

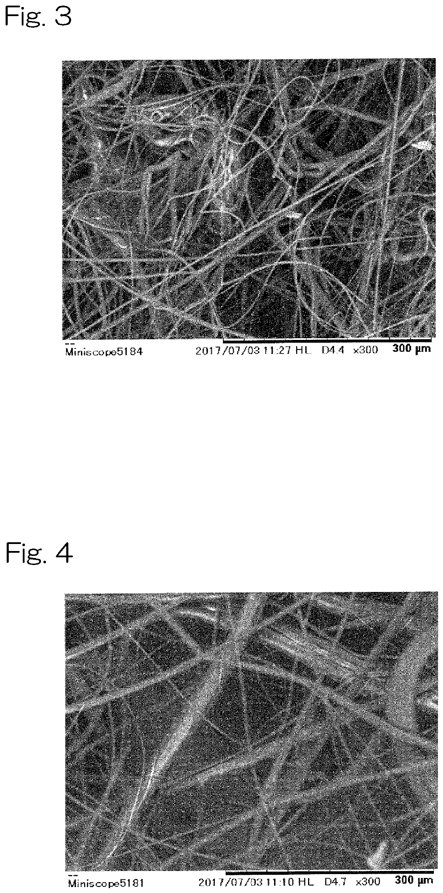

The same operation as in Example 1 was carried out, except that the nozzle single hole diameter (diameter) was set to 0.3 mm. The obtained melt-blown nonwoven fabric has a basis weight of 31.8 g/m² and a thickness of 0.608 mm, and a magnified electron micrograph of the obtained melt-blown nonwoven fabric is shown in Fig. 3.

Example 3

The same operation as in Example 2 was carried out, except that the heating temperature in the supply portion was set to 350°C, the kneading conditions in the kneading portion were under heating at 360°C and a screw rotation speed (N) of 10.5 rpm, and discharge from the nozzle was performed at a spinning temperature of 360°C in a discharge amount (Q) of 4.3 kg/hr as spinning conditions. The viscosity ($\eta$) of the resin melt introduced to the kneading portion was 150 poise. The obtained melt-blown nonwoven fabric has a basis weight of 31.2 g/m² and a thickness of 0.612 mm, and a magnified electron micrograph of the obtained melt-blown nonwoven fabric is shown in Fig. 4.

Example 4

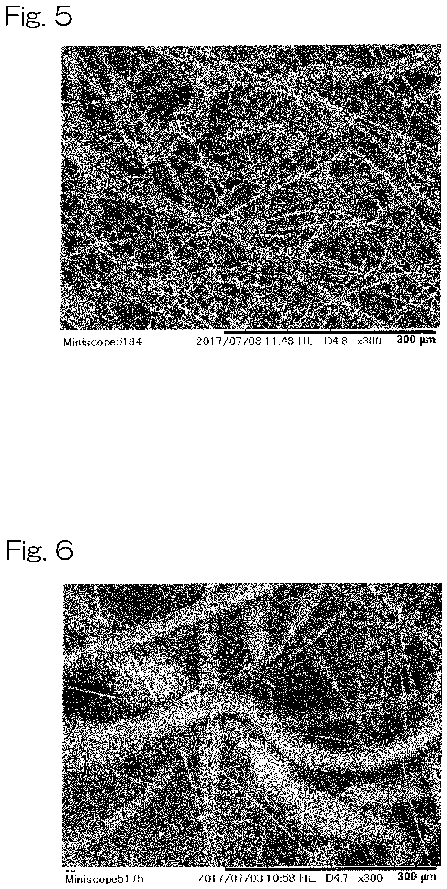

The same operation as in Example 2 was carried out, except that the used resin was an ethylene-norbornene copolymer (trade name "TOPAS" 6013, manufactured by Polyplastics Co., Ltd.) having a norbornene content of 75 mass%. The viscosity ($\eta$) of the resin melt introduced to the kneading portion was 180 poise. The obtained melt-blown nonwoven fabric has a basis weight of 20.7 g/m$^2$ and a thickness of 0.286 mm, and a magnified electron micrograph of the obtained melt-blown nonwoven fabric is shown in Fig. 5.

Comparative Example 1

The same operation as in Example 2 was carried out, except that the heating temperature in the supply portion was set to 300°C, the kneading conditions in the kneading portion were under heating at 320°C and a screw rotation speed (N) of 10.8 rpm, and discharge from the nozzle was performed at a spinning temperature of 320°C in a discharge amount (Q) of 4.8 kg/hr as spinning conditions. The viscosity ($\eta$) of the resin melt introduced to the kneading portion was 270 poise. The obtained melt-blown nonwoven fabric has a basis weight of 70.3 g/m$^2$ and a thickness of 1.154 mm, and a magnified electron micrograph of the obtained melt-blown nonwoven fabric is shown in Fig. 6.

[Table 1]

| | | Ex. 1 | Ex. 2 | Ex. 3 | Ex. 4 | Com. Ex. 1 |
|---|---|---|---|---|---|---|
| Production conditions | Nozzle hole diameter [mm] | 0.15 | 0.3 | 0.3 | 0.3 | 0.3 |
| | $\eta$ [poise] | 100 | 100 | 150 | 180 | 270 |
| | N [rpm] | 5.0 | 5.0 | 10.5 | 5.0 | 10.8 |
| | Q [kg/hr] | 2.2 | 2.2 | 4.3 | 2.2 | 4.8 |
| | $(Q\times\eta)/N$ | 43.3 | 43.3 | 61.9 | 78.0 | 120.3 |
| | Spinning temperature [°C] | 380 | 380 | 360 | 380 | 320 |
| MB nonwoven fabric | Basis weight [g/m$^2$] | 34.2 | 31.8 | 31.2 | 20.7 | 70.3 |
| | Thickness A [mm] | 0.430 | 0.608 | 0.612 | 0.286 | 1.154 |
| | Air permeability B [cm$^3$/cm$^2$·s] | 45 | 131 | 472 | 107 | 670 |
| | B/A | 105 | 215 | 771 | 374 | 581 |
| | Average fiber diameter [$\mu$m] | 2.8 | 7.2 | 11.8 | 6.1 | 14.8 |
| | CV value [%] | 101.0 | 88.0 | 100.8 | 84.0 | 110.6 |
| | Average fiber diameter Dc [$\mu$m] | 2.3 | 5.4 | 8.3 | 4.8 | 8.9 |
| | Average fiber diameter Db [$\mu$m] | 5.3 | 15.1 | 27.3 | 12.5 | 37.3 |

As shown in Table 1, in Comparative Example 1, since both the viscosity of the resin melt introduced to the kneading portion and the value of $(Q\times\eta)/N$ were high, the cyclic olefinic resin in the kneading portion were affected by mechanical energy, so that many thick fibers derived from mechanochemical reaction existed (Fig. 6). Due to intermingling of the thick fibers, in the MB nonwoven fabric of Comparative Example 1, the average fiber diameter as well as the average fiber diameter Db of fibers larger than the third quartile were both larger than those of the MB nonwoven fabrics of Examples 1 to 4. In addition, due to intermingling of the thick fibers, the air permeability was larger than those of Examples 1 to 4, and thus the denseness of the fabric was not sufficient.

On the contrary, in each of Examples 1 to 4, since the viscosity of the resin melt introduced to the kneading portion was controlled to be lower, generation of thick fibers derived from mechanochemical reaction was suppressed (Figs. 2 to 5). Therefore, in the MB nonwoven fabrics of Examples 1 to 4, the average fiber diameter as well as the average fiber diameter Db of fibers larger than the third quartile were both smaller than those of Comparative Example 1.

Moreover, the obtained MB nonwoven fabrics had lower air permeabilities than that of Comparative Example 1, and thus the denseness thereof were enhanced. Furthermore, the MB nonwoven fabrics of Examples 1, 2, and 4 had small air permeability per thickness (B/A) and were particularly excellent in denseness. In particular, each of the MB nonwoven fabrics of Examples 2 and 4 was pleasant to touch, and the reason for this may be that the CV value of the average fiber diameter of the fibers constituting the MB nonwoven fabric was reduced to 90% or less.

Example 5

Charging treatment was performed by a corona discharge method on the melt-blown nonwoven fabric obtained in Example 2. The specific conditions of the corona discharge method are as follows. The collection efficiency (%) and the pressure loss (Pa) of the obtained charged nonwoven fabric were measured in accordance with the above-described method. The results are shown in Table 2.

• Used power supply: DC high voltage stabilized power supply
• Linear distance between electrodes to which voltage is applied: 35 mm
• Applied voltage: -28 kV
• Applied current: 8.0 mA
• Temperature: 25°C
• Treatment time: 1 second

Example 6

A charged nonwoven fabric was obtained in the same manner as in Example 5 except that the melt-blown nonwoven fabric obtained in Example 4 was used instead of the melt-blown nonwoven fabric obtained in Example 2. The collection efficiency (%) and the pressure loss (Pa) of the obtained charged nonwoven fabric were measured in accordance with the above-described method. The results are shown in Table 2.

[Table 2]

|  | Ex. 5 | Ex. 6 |
|---|---|---|
| Collection efficiency [%] | 96.4 | 89.0 |
| Pressure loss [Pa] | 9 | 7 |

The charged nonwoven fabrics obtained in Examples 5 and 6 have a collection efficiency of equal to or greater than 85% and a pressure loss of equal to or less than 10 Pa, and thus are suitable for use for various filter applications.

INDUSTRIAL APPLICABILITY

The MB nonwoven fabric according to the present invention can be used for various applications such as medical, beauty, and hygiene materials, industrial materials, daily commodities, and clothing as described above. In particular, the MB nonwoven fabric according to the present invention is suitable for use for a filter for filtrating a liquid and an air filter (for example, an air filter for a clean room), a transdermal administration sheet (for example, a transdermal absorption preparation or a skin patch), etc., since the MB nonwoven fabric according to the present invention is excellent in denseness and low elution of impurities.

Preferred embodiments of the present invention are shown and described with reference to the accompanying drawings. It is to be understood that various changes, modifications and omissions may be made without departing from the spirit of the present invention and are encompassed in the scope of the claims.

Reference Numerals

10...extruder
12...hopper
20...screw
22...supply portion
24...kneading portion
26...compressing portion
28...weighing portion
30...cylinder
40...die
42...nozzle
50... discharged fibrous material

60...collecting member
62...collection surface

**Claims**

1. A production method of a melt-blown nonwoven fabric containing a cyclic olefinic resin, the production method including at least:

   a melting step of melting a resin composition containing at least the cyclic olefinic resin by heating in a supply portion to obtain a resin melt;
   a kneading step of introducing the resin melt to a kneading portion and kneading the resin melt in the kneading portion by rotation of a screw under heating to obtain a resin-kneaded material;
   a discharging step of discharging the resin-kneaded material with air from a nozzle; and
   a collecting step of collecting a fibrous material discharged from the nozzle on a collection surface to obtain a web, wherein the resin melt introduced to the kneading portion has a viscosity $\eta$ of 8 to 20 Pa·s (80 to 200 poise) under the condition of shear rate r = 1200 sec$^{-1}$ at the temperature in the supply portion.

2. The production method of the melt-blown nonwoven fabric according to claim 1, wherein the following formula (2) is satisfied,

$$20 \le (Q \times \eta)/N \le 100 \quad (2),$$

   wherein Q represents a discharge amount (kg/hr) of the resin-kneaded material from the nozzle, $\eta$ represents the viscosity (poise) of the resin melt introduced to the kneading portion, and N represents a screw rotation speed (rpm).

3. The production method of the melt-blown nonwoven fabric according to claim 1 or 2, wherein a heating temperature in the supply portion is 300 to 380°C.

4. A melt-blown nonwoven fabric produced by the method recited in any one of claims 1 to 3, the melt-blown nonwoven fabric including fibers containing a cyclic olefinic resin, wherein, among fiber diameter data at 100 points that are randomly selected from an SEM image taken in a magnification of 1,000, and ordered from a smallest fiber diameter to a largest fiber diameter and divided into quartiles, an average value of fiber diameter data having a larger than a third quartile is equal to or less than 30 $\mu$m, and wherein a CV value of an average fiber diameter of the fibers is equal to or less than 110%, which is the ratio of the standard deviation of the fiber diameter data at 100 points with respect to the average fiber diameter.

5. The melt-blown nonwoven fabric according to claim 4, wherein the cyclic olefinic resin is a copolymer including at least norbornene units.

6. The melt-blown nonwoven fabric according to claim 4 or 5, wherein the melt-blown nonwoven fabric has an air permeability of 10 to 550 cm$^3$/cm$^2$·s measured in accordance with JIS L 1906.

7. The melt-blown nonwoven fabric according to any one of claims 4 to 6, wherein the melt-blown nonwoven fabric has a basis weight of 10 to 50 g/m$^2$.

8. The melt-blown nonwoven fabric according to any one of claims 4 to 7, wherein the melt-blown nonwoven fabric satisfies the following formula (1),

$$80 \le B/A \le 800 \quad (1):$$

   wherein A represents a thickness (mm), and B represents an air permeability (cm$^3$/cm$^2$·s).

9. The melt-blown nonwoven fabric according to any one of claims 4 to 8, wherein the melt-blown nonwoven fabric includes fibers having an average fiber diameter of 1 to 15 $\mu$m.

10. The melt-blown nonwoven fabric according to any one of claims 4 to 9, wherein a ratio Db/Dc of the average value (Db) with respect to an average value (Dc) is equal to or less than 4.0, wherein Db is the average value of fiber diameter data having a fiber diameter larger than a third quartile, and Dc is an average value of fiber diameter data having a fiber diameter larger than a first quartile and smaller than the third quartile.

11. A filter in which the melt-blown nonwoven fabric as recited in any one of claims 4 to 10 is used.

12. A support for a transdermal administration sheet releasing an active ingredient, wherein the support is composed of the melt-blown nonwoven fabric as recited in any one of claims 4 to 10.

13. A transdermal administration sheet including at least an active ingredient and the support as recited in claim 12.

**Patentansprüche**

1. Verfahren zum Herstellen eines schmelzgeblasenen Vliesstoffs, der ein zyklisches Olefinharz enthält, wobei das Herstellungsverfahren mindestens aufweist:

   einen Schmelzschritt des Schmelzens einer Harzzusammensetzung, die mindestens das zyklische Olefinharz enthält, durch Erhitzen in einem Zufuhrabschnitt, um eine Harzschmelze zu erhalten;
   einen Knetschritt des Einführens der Harzschmelze in einen Knetabschnitt und des Knetens der Harzschmelze im Knetabschnitt durch Drehen einer Schnecke unter Erwärmung, um ein harzgeknetetes Material zu erhalten;
   einen Abgabeschritt des Abgebens des harzgekneteten Materials mit Luft aus einer Düse; und
   einen Sammelschritt des Sammelns eines Fasermaterials, das aus der Düse abgegeben wird, auf einer Sammelfläche, um eine Bahn zu erhalten,
   wobei die in den Knetabschnitt eingeführte Harzschmelze eine Viskosität $\eta$ von 8 bis 20 Pa·s (80 bis 200 Poise) unter der Bedingung einer Scherrate r = 1200 s$^{-1}$ bei der Temperatur im Zufuhrabschnitt aufweist.

2. Verfahren zur Herstellung eines schmelzgeblasenen Vliesstoffs nach Anspruch 1, wobei die folgende Formel (2) erfüllt ist,

$$20 \leq (Q \times \eta)/N \leq 100 \ (2),$$

   wobei Q eine Abgabemenge (kg/h) des harzgekneteten Materials aus der Düse darstellt, $\eta$ die Viskosität (Poise) der in den Knetabschnitt eingeführten Harzschmelze darstellt und N eine Schneckendrehzahl (U/min) darstellt.

3. Verfahren zur Herstellung eines schmelzgeblasenen Vliesstoffs nach Anspruch 1 oder 2, wobei die Heiztemperatur im Zufuhrabschnitt 300 bis 380 °C beträgt.

4. Schmelzgeblasener Vliesstoff, hergestellt nach dem Verfahren gemäß einem der Ansprüche 1 bis 3, wobei der schmelzgeblasene Vliesstoff Fasern enthält, die ein zyklisches Olefinharz enthalten, wobei unter den Faserdurchmesserdaten an 100 Punkten, die zufällig aus einem REM-Bild ausgewählt sind, das mit einer Vergrößerung von 1000 aufgenommen ist, und von einem kleinsten Faserdurchmesser zu einem größten Faserdurchmesser geordnet und in Quartile unterteilt sind, ein Durchschnittswert der Faserdurchmesserdaten, die größer als ein drittes Quartil sind, gleich oder kleiner als 30 $\mu$m ist, und wobei ein CV-Wert eines durchschnittlichen Faserdurchmessers der Fasern gleich oder kleiner als 110 % ist, was das Verhältnis der Standardabweichung der Faserdurchmesserdaten an 100 Punkten in Bezug auf den durchschnittlichen Faserdurchmesser ist.

5. Schmelzgeblasener Vliesstoff nach Anspruch 4, wobei das zyklische Olefinharz ein Copolymer ist, das mindestens Norborneneinheiten enthält.

6. Schmelzgeblasener Vliesstoff nach Anspruch 4 oder 5, wobei der schmelzgeblasene Vliesstoff eine Luftdurchlässigkeit von 10 bis 550 cm$^3$/cm$^2$·s, gemessen nach JIS L 1906, aufweist.

7. Schmelzgeblasener Vliesstoff nach einem der Ansprüche 4 bis 6, wobei der schmelzgeblasene Vliesstoff ein Flächengewicht von 10 bis 50 g/m$^2$ aufweist.

8. Schmelzgeblasener Vliesstoff nach einem der Ansprüche 4 bis 7, wobei der schmelzgeblasene Vliesstoff die folgende Formel (1) erfüllt,

$$80 \leq B/A \leq 800 \qquad (1),$$

wobei A eine Dicke (mm) und B eine Luftdurchlässigkeit ($cm^3/cm^2{\cdot}s$) repräsentiert.

9. Schmelzgeblasener Vliesstoff nach einem der Ansprüche 4 bis 8, wobei der schmelzgeblasene Vliesstoff Fasern mit einem durchschnittlichen Faserdurchmesser von 1 bis 15 $\mu$m enthält.

10. Schmelzgeblasener Vliesstoff nach einem der Ansprüche 4 bis 9, wobei ein Verhältnis Db/Dc des Durchschnittswerts (Db) in Bezug auf einen Durchschnittswert (Dc) gleich oder kleiner als 4,0 ist, wobei Db der Durchschnittswert von Faserdurchmesserdaten mit einem Faserdurchmesser größer als ein drittes Quartil ist, und Dc ein Durchschnittswert von Faserdurchmesserdaten mit einem Faserdurchmesser größer als ein erstes Quartil und kleiner als das dritte Quartil ist.

11. Filter, in dem der schmelzgeblasene Vliesstoff nach einem der Ansprüche 4 bis 10 verwendet wird.

12. Träger für ein transdermales Verabreichungsblatt, das einen Wirkstoff freisetzt, wobei der Träger aus dem schmelzgeblasenen Vliesstoff nach einem der Ansprüche 4 bis 10 besteht.

13. Transdermales Verabreichungsblatt, das mindestens einen Wirkstoff und den in Anspruch 12 genannten Träger enthält.

**Revendications**

1. Procédé de production d'un tissu non-tissé obtenu par fusion-soufflage contenant une résine à base d'oléfine cyclique, le procédé de production incluant au moins :

   une étape de fusion consistant à faire fondre une composition de résine contenant au moins la résine à base d'oléfine cyclique par chauffage dans une partie d'alimentation pour obtenir une résine fondue ;
   une étape de malaxage consistant à introduire la résine fondue dans une partie de malaxage et à malaxer la résine fondue dans la partie de malaxage par rotation d'une vis sous chauffage pour obtenir un matériau malaxé de résine ;
   une étape de décharge consistant à décharger le matériau malaxé de résine avec de l'air par une buse ; et
   une étape de collecte consistant à collecter un matériau fibreux déchargé par la buse sur une surface de collecte pour obtenir une toile,

   dans lequel la résine fondue introduite dans la partie de malaxage a une viscosité $\eta$ de 8 à 20 Pa.s (80 à 200 poises) sous la condition d'un taux de cisaillement r = 1200 sec$^{-1}$ à la température dans la partie d'alimentation.

2. Procédé de production du tissu non tissé obtenu par fusion-soufflage selon la revendication 1, dans lequel la formule (2) suivante est satisfaite,

$$20 \leq (Q \times \eta)/N \leq 100 \qquad (2),$$

   dans laquelle Q représente une quantité de décharge (kg/h) du matériau malaxé de résine par la buse, $\eta$ représente la viscosité (poise) de la résine fondue introduite dans la partie malaxage, et N représente une vitesse de rotation de vis (tr/m).

3. Procédé de production du tissu non tissé obtenu par fusion-soufflage selon la revendication 1 ou 2, dans lequel une température de chauffage dans la partie d'alimentation est de 300 à 380 °C.

4. Tissu non tissé obtenu par fusion-soufflage produit par le procédé selon l'une quelconque des revendications 1 à 3, le tissu non tissé obtenu par fusion-soufflage incluant des fibres contenant une résine à base d'oléfine cyclique, dans lequel, parmi des données de diamètres de fibre à 100 points qui sont sélectionnés de manière aléatoire sur une image MEB prise à un agrandissement de 1000, et ordonnées d'un diamètre de fibre le plus petit à un diamètre de fibre le plus grand et divisées en quartiles, une valeur moyenne de données de diamètres de fibre supérieurs à un troisième quartile est inférieure ou égale à 30 $\mu$m, et dans lequel une valeur CV d'un diamètre de fibre moyen des fibres est inférieure ou égale à 110 %, ce qui est le rapport de l'écart type des données de diamètres de fibre à 100 points par rapport au diamètre de fibre moyen.

5. Tissu non tissé obtenu par fusion-soufflage selon la revendication 4, dans lequel la résine à base d'oléfine cyclique est un copolymère incluant au moins des unités norbornène.

6. Tissu non tissé obtenu par fusion-soufflage selon la revendication 4 ou 5, dans lequel le tissu non tissé obtenu par fusion soufflage a une perméabilité à l'air de 10 à 550 cm$^3$/cm$^2$.s mesurée conformément à JIS L 1906.

7. Tissu non tissé obtenu par fusion-soufflage selon l'une quelconque des revendications 4 à 6, dans lequel le tissu non tissé obtenu par fusion-soufflage a une masse surfacique de 10 à 50 g/m$^2$.

8. Tissu non tissé obtenu par fusion-soufflage selon l'une quelconque des revendications 4 à 7, dans lequel le tissu non tissé obtenu par fusion-soufflage satisfait à la formule (1) suivante,

$$80 \leq B/A \leq 800 \qquad (1) :$$

dans laquelle A représente une épaisseur (mm), et B représente une perméabilité à l'air (cm$^3$/cm$^2$.s).

9. Tissu non tissé obtenu par fusion-soufflage selon l'une quelconque des revendications 4 à 8, dans lequel le tissu non tissé obtenu par fusion-soufflage inclut des fibres ayant un diamètre de fibre moyen de 1 à 15 $\mu$m.

10. Tissu non tissé obtenu par fusion-soufflage selon l'une quelconque des revendications 4 à 9, dans lequel un rapport Db/Dc de la valeur moyenne (Db) par rapport à une valeur moyenne (Dc) est inférieur ou égal à 4,0, dans lequel Db est la valeur moyenne des données de diamètres de fibre ayant un diamètre de fibre supérieur à un troisième quartile, et Dc est une valeur moyenne de données de diamètres de fibre ayant un diamètre de fibre supérieur à un premier quartile et inférieur au troisième quartile.

11. Filtre dans lequel le tissu non tissé obtenu par fusion-soufflage selon l'une quelconque des revendications 4 à 10 est utilisé.

12. Support pour feuille d'administration transdermique libérant un principe actif, dans lequel le support se compose du tissu non tissé obtenu par fusion-soufflage selon l'une quelconque des revendications 4 à 10.

13. Feuille d'administration transdermique incluant au moins un principe actif et le support selon la revendication 12.

## Fig. 1

## Fig. 2

Miniscope5205    2017/07/03 12:04 HL  D4.3  x300  **300 μm**

## Fig. 3

Miniscope5184    2017/07/03 11:27 HL  D4.4  x300    300 µm

## Fig. 4

Miniscope5181    2017/07/03 11:10 HL  D4.7  x300    300 µm

Fig. 5

Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001210549 A
- WO 0102635 A1
- US 20110259818 A